(19) Europäisches Patentamt
European Patent Office
Office européen des brevets

(11) Publication number: **0 315 106 B1**

# (12) EUROPEAN PATENT SPECIFICATION

(45) Date of publication of patent specification: **01.07.92**

(51) Int. Cl.[5]: **A61L 11/00**, //A01N59/00

(21) Application number: **88118123.4**

(22) Date of filing: **31.10.88**

## (54) Method for sterilizing solid refuse by active chlorine released by the reaction of halazone with water.

(30) Priority: **06.11.87 IT 2253487**

(43) Date of publication of application:
**10.05.89 Bulletin 89/19**

(45) Publication of the grant of the patent:
**01.07.92 Bulletin 92/27**

(84) Designated Contracting States:
**AT BE CH DE ES FR GB GR LI LU NL SE**

(56) References cited:
**EP-A- 0 196 151**

**CHEMICAL ABSTRACTS, vol. 83, no. 12, 22nd September 1975, page 358, abstract no. 103106g, Columbus, Ohio, US; & JP-A-74 30 940 (K. NOZAK) 17-08-1974**

(73) Proprietor: **L. MOLTENI & C. DEI F.LLI ALITTI S.P.A**
**Strada Statale, 67 Loc. Granatieri**
**I-50018 Scandicci Firenze(IT)**

(72) Inventor: **Gasparotti, Franca Angela**
**Via G. Mameli, 20**
**Firenze(IT)**

(74) Representative: **Gervasi, Gemma**
**NOTARBARTOLO & GERVASI Srl Viale Bianca Maria 33**
**I-20122 Milan(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

### Field of the invention

This invention relates to a method for disinfecting solid refuse contained in bags by chlorine which is released in the gaseous state by the reaction of halazone with an alkaline solution, and to the relative container.

### Prior Art

Halazone and mixtures containing it are known for their use in disinfecting drinking water.

Halazone, or 4-(dichlorosulphamoyl) benzoic acid, decomposes on contact with water to produce hypochlorous acid, which exerts its known bactericide action in the water by releasing active chlorine.

Halazone is however poorly soluble in water but it is well known that its solubility can be increased by mixing it with an alkaline substance such as sodium carbonate, which salifies the carboxyl function.

Disinfection is also required for solid refuse originating from hospitals, biological laboratories and the like, which has to be disinfected before being burnt. This operation is currently done using disinfectant liquids, which have drawbacks both from the operational viewpoint in that they are hardly practical, and also from the result viewpoint in that they are inefficient, because it is impossible to distribute the liquid uniformly throughout the solid refuse mass.

### Summary of the invention

This invention relates to a method for disinfecting solid refuse contained in bags by the action of chlorine released from solid mixtures containing halazone, characterised by:

a) bringing the solid mixture containing halazone into contact with an alkaline solution in a suitable reaction container which confines the aqueous suspension in such a manner as to prevent it escaping whatever its position;

b) immediately introducing said container into the bag in any position;

c) closing the bag to allow the chlorine released in the gaseous state by the reaction to exercise its disinfectant action.

The invention also relates to said reaction container, which is characterised by being provided with a tube which extends inwardly as a continuation of the neck portion in order to prevent escape of liquid whatever the position it assumes within the bag.

### Detailed description of the invention

The method of the present invention is based on the following reaction between halazone and water:

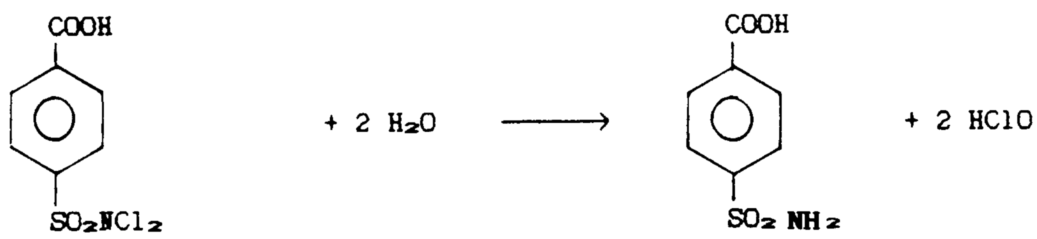

The hypochlorous acid then decomposes to release "active" chlorine which exercises its known bactericide action.

The expression "active chlorine content of a substance" conventionally means the quantity of molecular chlorine equivalent to the theoretically evolved hypochlorous acid. As one mole of halazone releases two moles of hypochlorous acid, its active chlorine content is 52%, this value being double the chlorine present in the molecule.

For the method according to the invention, a solid mixture of halazone and sodium carbonate is used having an active chlorine content of between 15 and 25% by weight and a sodium carbonate content of between 50 and 70% by weight. This mixture is used in powder form.

To release the active chlorine this mixture is brought into contact with an alkaline solution having a pH of between 11 and 13.

The weight ratio of the alkaline solution to the active chlorine contained in the solid mixture is between 15 and 25.

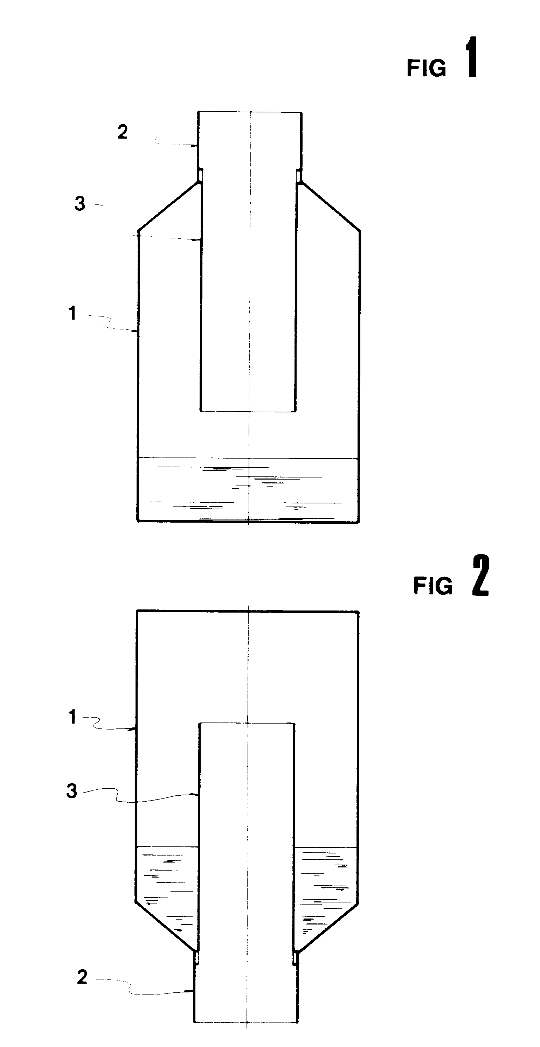

The contact between said solid mixture and said alkaline solution is made in the container shown in Figures 1 and 2 in which the reference numeral 1 indicates the cylindrical wall of the container, 2 indicates the cylindrical neck portion and 3 indicates a tube projecting inwards as a continuation of said neck portion and coaxial to said wall 1.

Contact between said solid mixture and said water is obtained by introducing the solid mixture into the illustrated container containing the alkaline solution.

By comparing Figure 1 with Figure 2 it can be seen that with this type of container the liquid can never escape whatever the position assumed by the container, be this vertical, inverted or inclined, whereas the produced gas escapes through the neck portion.

When said solid mixture and said solution are in mutual contact the container is introduced into the bag containing the solid refuse to be disinfected, and the bag is closed.

The released chlorine diffuses through the refuse to produce very efficient disinfecting. Thus the method and container according to the present invention allow solid refuse to be disinfected in a manner which obviates the drawbacks of the known art by virtue of its practicality, its reliability and its efficiency. The invention is particularly suitable for application to disinfecting solid refuse from hospitals, biological laboratories and the like.

The following procedure was carried out to demonstrate the effectiveness of the treatment.

Portions of clean or medicated gauze were superinfected by the operator with strains of bacteria and mycetes either from collections or isolated from patients, and were placed in three different positions, i.e.at the top, in the middle and at the bottom, of special refuse bags with a useful volume of 80 litres, originating from hospital departments. A reaction container of the type show in Figure 1 containing 7.5 g of solid halazone and sodium carbonate mixture and 30 ml of alkaline solution at pH 12 was then placed in each bag. The bag was immediately closed to prevent escape of gaseous chlorine.

A control was prepared in an analogous manner and immediately analyzed to evaluate the initial microbe charge.

After 24 hours of contact the treated gauze portions were removed from the bag to evaluate the numerical reduction in micro-organisms compared with the control material.

The results demonstrate strong activity of the chlorine gas against Gram-negative bacteria. In particular, three strains of Pseudomonas aeruginosa, multiresistant and nosocomial, and present in a quantity of $10^9-10^{12}$, were reduced to less than 10.

Klebsiella pneumoniae was also reduced after treatment from $1 \times 10^9$ to less than 10 in the samples arranged in the three positions within the bag.

The same result was obtained for Escherichia coli, which was reduced from $1.9 \times 10^{12}$ to less than 10 in all three samples.

The results were also excellent in the case of mycetes, in that Candida albicans, which was present in the control in a quantity of $1 \times 10^6$, was reduced to less than 10 in the three samples.

In the case of other strains which proved more resistant, such as coagulase-negative Staphylococcus, which was reduced on the average from $2 \times 10^{12}$ to $4 \times 10^3$, it was found that by doubling the halazone quantity, i.e.the quantity of available chlorine, by inserting two containers in the bag, an almost total germ reduction was obtained in the three samples.

The following examples relative to the release of the chlorine available in the halazone-sodium carbonate mixture are given by way of illustration.

EXAMPLE 1

0.75 g of a 2:3 w/w halazone-sodium carbonate mixture corresponding to 0.15 g of active chlorine were placed in a 25 ml container.

3 ml of water were then added and left to react for 90 minutes at ambient temperature.

The residual active chlorine was then determined, from which it was calculated that 78.2% of the active chlorine had been released.

EXAMPLES 2 - 4 (comparison)

Example 1 was repeated but with the difference that respectively 2, 5 and 7 ml of water were used, to

obtain released active chlorine quantities of 68.5%, 66.4% and 65.2% respectively.

EXAMPLE 5

Example 1 was repeated but with the difference that the water pH was adjusted to 12.8 by adding NaOH.

The active chlorine released was 71.2%.

EXAMPLE 6 (comparison)

Example 1 was repeated but with the difference that the water pH was adjusted to 1.2 by adding HCl. The active chlorine released was 71.2%.

EXAMPLES 7 - 8 (comparison)

Example 1 was repated but with the difference that the water pH was adjusted to 13 and 13.5 respectively by adding NaOH. The active chlorine released was 82.3% and 79.8% respectively.

**Claims**

1. A method for disinfecting solid refuse contained in bags by the action of active chlorine released from solid mixtures containing halazone, characterised by:
   a) bringing the solid mixture containing halazone into contact with an alkaline solution in a suitable reaction container which confines the aqueous suspension in such a manner as to prevent it escaping whatever its position;
   b) immediately introducing said container into the bag in any position;
   c) closing the bag to allow the chlorine released in the gaseous state by the reaction to exercise its disinfectant action.

2. A method as claimed in claim 1, characterised in that said solid mixture containing halazone consists of halazone and sodium carbonate, the active chlorine content being between 15 and 25% by weight and the sodium carbonate content being between 50 and 70 % by weight.

3. A method as claimed in claim 1, characterised in that said solid mixture containing halazone is used in powder form.

4. A method as claimed in claim 1, characterised in that said alkaline solution has a pH of between 11 and 13.

5. A method as claimed in claim 1, characterised in that the weight ratio of said alkaline solution to the active chlorine contained in said solid mixture is between 15 and 25.

6. A method as claimed in claim 1, characterised in that said reaction container is provided with a tube (3) which projects inwards as a continuation of the neck portion (2) and is coaxial to the container walls (1).

**Revendications**

1. Procédé de désinfection de déchets solides contenus dans des sacs par l'action de chlore réactif libéré à partir de mélanges solides contenant de l'halazone caractérisé par :
   a) la mise en contact du mélange solide contenant de l'halazone avec une solution basique dans un réservoir de réaction adapté qui enferme la suspension aqueuse de manière à l'empêcher de s'échapper quelque soit sa position;
   b) l'introduction immédiate dudit réservoir à l'intérieur du sac dans toute position;
   c) la fermeture du sec pour permettre la chlore libéré à l'état gazeux par la réaction d'exercer son pouvoir désinfectant.

2. Procédé selon la revendication 1, caractérisé en ce que ledit mélange solide contenant de l'halazone se compose d'halazone et de carbonate de sodium, la teneur en chlore réactif étant comprise entre 15 et

25 % en poids et la teneur du carbonate de sodium étant comprise entre 50 et 70 % en poids.

**3.** Procédé selon la revendication 1, caractérisé en ce que ledit mélange solide contenant de l'halazone est utilisé sous forme de poudre.

**4.** procédé selon la revendication 1, caractérisé en ce que ladite solution basique a un pH compris entre 11 et 13.

**5.** Procédé selon la revendication 1, caractérisé en ce que le rapport de poids entre la solution basique et le chlore réactif contenu dans ledit mélange solide est compris entre 15 et 25.

**6.** procédé selon la revendication 1, caractérisé en ce que ledit réservoir de réaction est doté d'un tube (3) qui s'étend à l'intérieur comme un prolongement de la partie goulot (2) et qui est coaxial aux parois du réservoir.

**Patentansprüche**

**1.** Verfahren zum Desinfizieren von in Säcken enthaltenem festen Abfall durch die Wirkung von Chlor, das aus festen halazonhaltigen Mischungen freigesetzt wird, dadurch gekennzeichnet, daß

a) die halazonhaltige feste Mischung mit einer alkalischen Lösung in einem geeigneten Reaktionsbehälter in Berührung gebracht wird, der die wässerige Lösung auf solche Weise einschließt, daß ihr Entweichen, wie immer die Lage ist, verhindert wird,

b) der Behälter sofort in den Sack in jeder Stellung eingebracht wird,

c) der Sack verschlossen wird, um zu ermöglichen, daß das durch die Reaktion im gasförmigen Zustand freigesetzte Chlor seine desinfizierende Wirkung ausübt.

**2.** Verfahren nach Anspruch 1, dadurch gekennzeichnet, daß die halazonhaltige feste Mischung aus Halazon und Natriumcarbonat besteht, wobei der Gehalt an aktivem Chlor 15 bis 25 Gew.% und der Natriumcarbonatgehalt 50 bis 70 Gew.% beträgt.

**3.** Verfahren nach Anspruch 1, dadurch gekennzeichnet, daß die halazonhaltige feste Mischung in Pulverform verwendet wird.

**4.** Verfahren nach Anspruch 1, dadurch gekennzeichnet, daß die alkalische Lösung einen pH von 11 bis 13 aufweist.

**5.** Verfahren nach Anspruch 1, dadurch gekennzeichnet, daß das Gewichtsverhältnis der alkalischen Lösung zu dem in der festen Mischung enthaltenen aktiven Chlor 15 bis 25 beträgt.

**6.** Verfahren nach Anspruch 1, dadurch gekennzeichnet, daß der Reaktionsbehälter mit einem Rohr (3) versehen ist, das sich als Fortsetzung des Halsteiles (2) nach innen erstreckt und zu den Behälterwänden (1) koaxial ist.

FIG 1

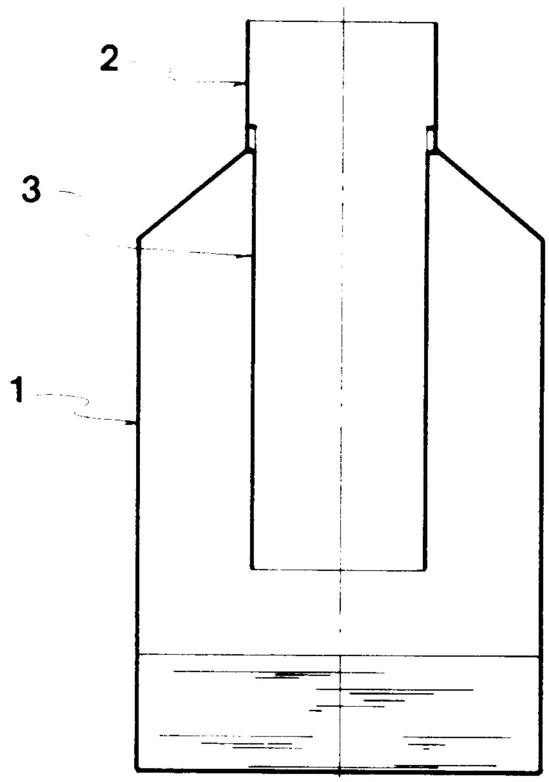


FIG 2